# EUROPEAN PATENT SPECIFICATION

(11) **EP 3 756 698 B1**
(45) Date of publication and mention of the grant of the patent: **30.04.2025**
(21) Application number: 20182782.1
(22) Date of filing: 29.06.2020
(51) Int. Cl.: A61L 24/10

(54) **A TISSUE SEALANT FOR USE IN ANTERIOR CRUCIATE LIGAMENT (ACL) RECONSTRUCTION**
GEWEBEVERSIEGELUNGSMITTEL ZUR VERWENDUNG BEI DER REKONSTRUKTION DES VORDEREN KREUZBANDES
AGENT DE SCELLEMENT TISSULAIRE DESTINÉ À ÊTRE UTILISÉ DANS LA RECONSTRUCTION DE LIGAMENTS CROISÉS ANTÉRIEURS (LCA)

(30) Priority: 28.06.2019 DK PA201970413
(43) Date of publication of application: 30.12.2020
(73) Proprietor: Vivostat A/S, 3450 Allerød (DK)
(72) Inventor: LANGE, Sven, 3520 Farum (DK)
(74) Representative: Nordic Patent Service A/S

(56) References cited:
- WO-A1-2019/007469
- MASAHIKO KEMMOCHI ET AL: "The use of platelet-rich fibrin with platelet-rich plasma support meniscal repair surgery", JOURNAL OF ORTHOPAEDICS, vol. 15, no. 2, 1 June 2018 (2018-06-01), pages 711 - 720, XP055753356, ISSN: 0972-978X, DOI: 10.1016/j.jor.2018.05.006

## Description

### TECHNICAL FIELD

The disclosure relates to a tissue sealant for use in Anterior Cruciate Ligament (ACL) reconstruction and more particularly to a fibrin sealant capable of promoting the graft-bone integration and ligamentization.

### BACKGROUND

Anterior Cruciate Ligament (ACL) reconstruction with autologous hamstring tendons is a proven method to restore the normal biomechanics and stability of the knee. The primary goal of ACL reconstruction is to create a stable knee with good biomechanical properties. A successful outcome depends on the healing quality of the reconstructed graft.

ACL reconstruction with semitendinosus graft is generally regarded to be a safe and reliable process yielding high stability if performed with the correct surgical technique and if a solid bone-graft integration is achieved.

Stability of the implanted graft depends on the integration and ligamentization period of the graft, which may be expected to last at least 4 to 6 months after the reconstruction. The integration and ligamentization periods generally comprise the production of type I collagen by fibroblasts to secure the integration and appearance of Sharpey fibers, to strengthen the ligamentization, and to provide a safe integration of the graft with the bone tunnels, so that the graft's biomechanical and structural properties resemble that of an intact ACL.

CN106474550A relates to a reconstruction method of a muscle tendon-bone tissue osseous complex. A biological preparation (osteoinductive composite) is prepared using Bone Matrix Protein (BMP) and fibrin gel, which is used in the healing of tendon-bone tissue. The suggested treatment procedure promotes the formation of bone tissue in the transplanted tendon (internal and external) in a bone tunnel, and crosslinks with the host-bone tissue (tunnel) to construct a new tendon-bone tissue complex. BMP was used as a raw material and fully dissolved under specific conditions to prepare a bone-inducing stock solution having a final concentration of 0.8-1.0 g/L. Fibrin is used as a glue carrier.

WO17151674 A1 discloses a method of endogenous stem cell activation for tendon/ligament osteintegration. By first recruiting endogenous stem cells to the site of injury, BMPs are then delivered in vivo to promote repair. Significant acceleration of healing via the above methods and compositions leads to fast recovery and return to normal activities. Enhanced tendon allograft integration into bone in a large animal model is shown.

WO16054687 A1 discloses a sutureless method of repairing ACL. The method includes the use of a collagen-containing patch for forming a bioactive chamber and then adhering the patch to the tissue. It is preferred to use laser emitted optical energy or radio frequency energy to "weld" the tissue but also fibrin glue may be used.

WO2010030714 (A2) provides compositions and methods for treatment of tendon and ligament injuries and/or repair of damaged tendons and ligament. The application discloses compositions comprising a biocompatible matrix and platelet-derived growth factor (PDGF). The biocompatible matrix comprises collagen and glycosaminoglycan, such as chondroitin sulfate.

Am J Sports Med. 2019 Jun 5 (doi: 10.1177/0363546519852616) reports that PRP provides no benefit for meniscal repair with ACL reconstruction. The study entailed 550 patients who underwent meniscal repair surgery with or without PRP concurrent ACL reconstruction. The independent effect of PRP on the risk of meniscal repair failure was determined by multivariate Cox proportional hazards modelling with adjustment for age, sex, body mass index, ACL status, tear pattern, tear vascularity, repair technique, side (medial or lateral), and number of sutures or implants used.

J. Orthop. 2018 May 15; 15(2):711-720 reports the use of platelet-rich fibrin (PRF) with platelet-rich plasma (PRP) support for meniscal repair surgery. 17 patients underwent arthroscopic meniscus repair with PRF and PRP using a novel device for the injection of PRF into the joint. Compared to a control group that did not receive PRF and PRP, there was no significant differences in the postoperative score.

Furthermore, follow-up MRI finding did not clearly show improvements.

Knee Surg. Sports Traumatol. Arthrosc (2015) 23:3614-3622 discloses the use of platelet rich fibrin matrix (PRFM) in ACL reconstruction. The PRFM is used as augmentation for ACL reconstruction in terms of graft-bone integration and knee stability. A statistically significant different was not detected between the treated group and the control group in terms of MRI and objective clinical evaluation.

MASAHIKO KEMMOCHI et al., JOURNAL OF ORTHOPAEDICS, vol. 15, no. 2, 2018, pages 711-720, discloses the use of platelet-rich fibrin with platelet-rich plasma for supporting meniscal repair surgery.

The prior art documents referred to above do not provide an effective procedure of ACL reconstruction. Therefore, an unmet need for relatively fast graft maturation and healing exist. Furthermore, it is desirable with ACL reconstruction procedures showing less side-effects, such as few incidences of postoperative hemarthrosis and excess synovial fluid.

### SUMMARY

The present invention relates to a tissue sealant for use in an Anterior Cruciate Ligament (ACL) reconstruction as defined in the claims. Thus, the present invention relates to a tissue sealant for use in an Anterior Cruciate Ligament (ACL) reconstruction, wherein a tunnel in femur and a tunnel in tibia are drilled and a tendon graft is accommodated in the femoral tunnel and the tibial tunnel for connecting the femur and the tibia bones together, the tissue sealant being provided on at least a part of the surface of the tendon graft, said tissue sealant comprising 8 mg/ml or more fibrin and at least one human or recombinant growth factor and/or platelets, and wherein at least a portion of the fibrin is fibrin I monomer at a pH lower than pH 5.

Surprisingly, the present invention shows fast maturation of the tendon graft as indicated by the signal intensity of MRI measurements recorded at the end of the post-operative 5^{th} month. Furthermore, low occurrence of side effects was noted, such as the percentage of hemarthrosis needed to be aspired and synovial fluid at the graft-tunnel interface. Any references to treatment by surgery or therapy are not part of the invention.

ACL reconstruction is a surgical tissue graft replacement of the anterior cruciate ligament, located in the knee, to restore its function after an injury. The surgical procedure generally starts with removal of the torn ligament from the knee in an arthroscopic procedure. In a subsequent step tunnels are drilled through the femur (thigh bone) and the tibia (shin bone) for accommodating the tendon graft. Then a tendon graft is connecting the femur and the tibia by pulling the tendon graft through the femoral and the tibial tunnels and fastening the tendon graft to the femur and the tibia, respectively. In certain embodiments of the ACL procedure it is suitable to prepare two or more tunnels in the femur and/or the tibia for accommodating two or more tendon grafts.

The tendon graft is generally an autograft, i.e. a tissue harvested from the patient's body. In some occasions it may be suitable to use allografts, i.e. tissue from another body, either a cadaver of a live donor. However, autografts are generally preferred because of the lower risk of graft-versus-host rejection.

The tendon graft is generally selected among the hamstring tendon, the patellar tendon, or the quadriceps tendon.

Hamstring tendons are made with the semitendinosus tendon, either alone or accompanied by the gracilis tendon for a stronger graft. The semitendinosus is an accessory hamstring (the primary hamstrings are left intact), and the gracilis is an accessory adductor (the primary adductors are left intact as well). The two tendons are commonly combined and referred to as a four-strand hamstring graft, made by a long piece (about 25 cm) removed from each tendon. The tendon segments may be folded and braided together to form a tendon of quadruple thickness for the graft. The braided segment is threaded through the tunnels of the tibia and femur, and its ends may be fixed with screws or similar fastening means on the opposite sides of the two bones.

The patellar tendon connects the patella (kneecap) to the tibia. The graft is normally taken from the injured knee, but in some circumstances, such as a second operation, the other knee may be used. Generally, the middle third of the tendon is used, with bone fragments removed on each end. The graft is then threaded through tunnels drilled in the tibia and femur and fastened into place. The patellar tendon is slightly larger than a hamstring graft.

The quadriceps tendon works with the quadriceps muscle to extend the leg. Part of the quadriceps may be removed and used as the tendon graft. In an embodiment of the invention the tendon graft is semitendinosus (ST) tendon graft due to lesser wound pain and decreased scar formation.

In a certain embodiment of the disclosure, the tendon graft is pulled through the tunnel and fixated by ACL TightRope^{®} RT implant obtainable from Arthrex. Other types of fixation of the graft tendon to the bones, such as screws, may also be used.

At the end of the ACL reconstruction, the knee may be emptied for any fluid present. While the tissue sealant may be applied to the surface of the tendon graft by a number of methods including brushing, injecting, pouring, etc. outside the body and/or inside the body it is generally preferred that the tissue sealant is sprayed on the surface of the graft. Application by spraying offers the possibility of blending or mixing a multi-component tissue sealant, e.g. a tissue sealant comprising 2 or more components, immediately before or after ejection from the nozzle of the application device.

In certain applications of the present invention it may be preferred to pretreat the tendon graft *in vitro* with the tissue sealant before insertion of the graft in the tunnels. After accommodation of the tendon graft in the tunnels, the tissue sealant may in a second treatment, be applied to the tendon graft to ensure a strong adherence of the tendon graft to the walls of the tunnels.

A convenient way of applying the tissue sealant to at least a part of the tendon graft is to convey the application device through one or both of the femoral tunnel and the tibial tunnel. Usually, the tissue sealant is applied "inside out" to reduce the risk of contamination, i.e. the delivery of the sealant starts in the part of the tunnel closest to the center of the knee and continues while the application device slowly is pulled out. Generally, it is preferred first to use the femoral tunnel for the introduction of the application device. In a certain embodiment of the disclosure the tissue sealant is conveyed in a lance through the femoral and/or tibial tunnels and then sprayed to the tendon-tunnel interface. In a certain aspect, the tissue sealant is further sprayed posterior to the posterior cruciate ligament (PCL) to avoid bleeding.

The present invention applies a concentration of fibrin in the tissue sealant, which is significantly higher than the concentration in plasma. The high content of fibrin provides a sticky tissue sealant that easily adheres to the walls of the femoral and/or the tibial tunnels. The tissue sealant further comprises one or more human or recombinant growth factor(s) and/or platelets. It is currently believed that the higher concentration of fibrin provides a sustained release matrix for accommodating growth factors and/or platelets. Over time, the fibrin matrix will be degraded by the digestion of naturally occurring enzymes like plasmin, thereby liberating one or more human or recombinant growth factor(s). The sustained release ensures that growth factors are provided to the area during an extended time period of the healing process.

The fibrin sealant is generally derived from mammal blood to form bovine fibrin, human fibrin, equine fibrin, porcine fibrin, etc. Human fibrin is preferably used to increase the biocompatibility. In a preferred aspect, the fibrin sealant is derived from the patient's own blood, i.e. the fibrin is autologous. The risk of mammal-borne contaminants is eliminated, which will protect the patient and the surgeon against e.g. viral diseases not yet identified. Furthermore, the use of autologous fibrin further increases the biocompatibility and reduces the risk of viral infections.

In a preferred embodiment, the tissue sealant contains one or more human or recombinant growth factor(s). The growth factors are capable of stimulating cell growth, proliferation, healing and cellular differentiation of the tissue, thereby promoting the healing process. Furthermore, the growth factors stimulate the ligamentization. The growth factors may emanate from a biological or synthetic origin. In a preferred embodiment, the human growth factors are derived from platelets.

Growth factors include platelet-derived growth factor, Transforming Growth Factor Beta (TGF-β), Insulin like Growth Factor (IGF), Fibroblast Growth Factor (FGF), and Vascular Endothelial Growth Factor (VEGF). These growth factors are expected to be present in every step of the healing process with the ability to activate the membrane receptors in integrines while enhancing the production of components of collagen matrices. Thus, the maturation and healing of the graft integration is regarded to be promoted by the presence of the growth factors.

Activated platelets release about 30 bioactive molecules that are mainly composed of growth factors and cytokines which promote tissue regeneration and soft-tissue maturation by stimulating mesenchymal cells, macrophages together with the osteoblasts. Activated platelets with the aforementioned capabilities are able to provide bone regeneration and soft-tissue maturation. It is currently believed that activated platelets accelerate tendon ligamentization leading to earlier return to daily activities and sports eventually.

In a preferred embodiment, the tissue sealant is enriched with platelets. The biological function of platelets is to help stop the bleeding by clumping and clotting blood vessel injuries. When performing an ACL reconstruction, the platelets present in the tissue sealant will increase the maturation at the graft-tunnel interface. The patient's own circulating platelets may also be attracted to the graft-tunnel interface and assist the platelets of the tissue sealant in the maturation and healing process. In a preferred aspect of the invention the human growth factors at least partly emanate from the platelets contained in the tissue sealant.

The platelets may be derived from a human blood source. In a preferred embodiment, the platelets of the tissue sealant are derived from the patient's own blood. It is generally believed that fewer complications are experienced with autologous platelets due to the absence or reduced risk of immunological reactions. In a certain embodiment the concentration of platelets in the tissue sealant is at least twice the concentration of platelets in the patient's own blood. The increased amount of platelets in the fibrin matrix provides sufficient growth factors during the sustained release period. In an embodiment of the invention the concentration of platelets in the tissue sealant is at least three times the concentration of platelets in the patient's own blood. Usually, the concentration of the platelets in the tissue sealant is no more than 10 times the concentration of platelets in the patient's own blood, such as no more than 7 times. In a certain aspect of the invention, the concentration of platelets in the tissue sealant is about 3 to about 9 times, such as about 5 to 7 times, the concentration of platelets in the patient's own blood.

At least a portion of the fibrin is fibrin I monomer maintained at a pH lower than pH 5. The advantage is that the fibrin I polymer is soluble under mild acid conditions and thus easily can be applied to the tendon graft. In a preferred embodiment, at least 90% of the fibrin in the tissue sealant is fibrin I.

According to the invention the amount of fibrin is at least 8 mg/mL. To ensure more suitable sustained-release matrix a higher concentration of fibrin I may be selected, such as a concentration above 10 mg/mL, such as above 12 mg/mL, such as above 14 mg/mL such as above 16 mg/mL. Suitably, the concentration of fibrin I does not exceed 50 mg/mL to reduce the risk of the sealant from becoming too viscous. Suitably, the concentration of the fibrin I does not exceed 40 mg/mL, such as 30 mg/mL. In a preferred embodiment of the invention the concentration of fibrin I in the tissue sealant is 15 to 25 mg/mL.

In certain embodiments of the present invention the tissue sealant may comprise an anti-fibrinolytic agent, such as tranexamic acid to reduce the decomposition of fibrin. Generally, the amount of tranexamic acid, when present is at least 0.05 mg/mL, such as at least a 0.1 mg/mL. The optional addition of anti-fibrinolytic agent such as tranexamic acid may be used to adjust the delivery time for the tissue factors. The time period of highest importance for obtaining a successful healing process is from day 0 to day 7. Thus, the amount of tranexamic acid may be adjusted so that a steady release of growth factors optionally derived from platelets is obtained. In a suitable embodiment of the invention, the amount of tranexamic acid in the tissue sealant of the invention is adjusted so that the recommended i.v. dose of 10 mg/kg 3-4 daily is not exceeded. Preferably, the concentration is not above 500 mg/ml in the sealant of the invention to prevent too slow degradation of the fibrin. At present, it is believed that a suitable concentration of anti-fibrinolytic agent is 1.5 mg/mL to 100 mg/mL.

In a preferred embodiment, part or all said fibrin I monomer after or simultaneously with application to the tendon graft is polymerized to fibrin II by activation with an alkaline agent. The advantage is that the fibrin II aggregates and forms a physical connection between the surface of the tendon graft and the interior walls of the femoral and tibial tunnel walls.

In a preferred aspect, the tissue sealant is Vivostat^{®} platelet enriched fibrin sealant, available under the trademark ArthroZheal^{™}. During the preparation of Vivostat^{®} platelet enriched fibrin sealant, citrate is transferred to the Vivostat^{®}-Preparation Unit, and 120 mL of blood is collected from the patient into the Preparation Unit. The pH 4 syringe is placed in the Preparation Unit, which is then loaded into the Vivostat^{®} Processor Unit. Upon activation of the automated process, the blood is warmed to 36°C and it is separated in the upper reservoir chamber of the Preparation Unit by centrifugation. Plasma (containing the platelet fraction) is then passed through three channels to the reaction (mixing) chamber of the Preparation Unit, and batroxobin that has been released from the cartridge contained in the Preparation Unit, is mixed with the separated platelet rich plasma. In the plasma mixture that contains acid-soluble fibrin I, the fibrin I polymerizes and is isolated on the walls of the chamber by repeated centrifugation. Excess fibrinogen and platelet depleted serum are then withdrawn back into the upper reservoir (waste) chamber of the Preparation Unit, leaving concentrated fibrin I and platelets in the reaction chamber. This platelet-rich fibrin concentrate is then mixed with 3.5 mL of pH 4 sodium acetate buffer to dissolve the fibrin I molecules. The resulting acidified platelet-rich fibrin I solution is transferred into the bottom of the Preparation Unit. Any residual pH 4 buffer is dumped into a separate bottom in the Preparation Unit and the acidified platelet-rich fibrin I solution is then withdrawn into the empty pH 4 buffer syringe.

The concentrated fibrin I monomer solution is stable for up to 8 hours at room temperature or for several days at -20°C. The fibrin I monomer containing the autologous platelets can be transferred to the desired applicator device for use in the present invention.

The concentrated fibrin I monomer solution is co-applied to the tendon graft with an alkaline buffer (carbonate/- bicarbonate buffer, pH 10) to activate the fibrin I. Thus, described herein is also a method for applying fibrin I to the tendon graft, comprising the step of spraying the tissue sealant of the invention comprising 8 mg/ml or more fibrin I to the tendon graft and simultaneously spraying an alkaline buffer to activate the fibrin I. The ratio between the concentrated fibrin I monomer solution and the alkaline buffer is usually in the range of 4-10:1. The co-spraying of the fibrin I solution and the alkaline buffer result in an almost instant polymerization of the fibrin I monomer to the polymer.

In a specific embodiment, to initiate the application process, both the carefully mixed fibrin syringe and the syringe containing pH 10 carbonate/bicarbonate buffer are loaded into the Applicator Unit. Upon activation of the device's priming sequence, the plungers of the syringes move upwards at a set rate to deliver the two solutions through a multi-lumen tube until the system is primed (i.e., both lumens are completely full from syringe to tip of the Spraypen Applicator). Upon further activation of the Spraypen Applicator, air, pressurized by the pump in the Application Unit, passes through a third lumen and exits together with the two solutions at the application tip where a fine spray is formed. Fibrin I in the now alkalized Vivostat^{®} platelet enriched fibrin ^{®} polymerizes, and after Vivostat^{®} platelet enriched fibrin is applied, fibrinopeptide B (FPB) is released and react with endogenous thrombin to form fibrin II, which is crosslinked and polymerized by the activity of endogenous FXIIIa. When the fibrin I is converted to the fibrin II the tendon graft will become attached to the walls of the femoral and/or the tibial tunnels.

The tissue sealant may be co-administered with a desired substance selected among drugs, structural reinforcing substances, cells, or a mixture thereof.

In an aspect of the invention, the desired substance for coadministration is an antibiotic agent, an antifibrinolytic agent, or a pain relief medicament. An antibiotic agent is generally used as a preventive treatment in case of contamination risk. An anti-fibrinolytic agent may be used to inhibit the fibrinolysis. A pain relief medicament may be used to increase the patient well-being or recovery. BMP (Bone Morphogenetic Protein) is a growth factor that also may be co-administered to promote the healing of the bones and/or the bone-graft integration.

In an embodiment of the invention the co-administered cells are stem cells, such as bone marrow or embryonic stem cells, or cells derived from adipose tissue. The co-administered cells may be useful in the formation of tissue at or around the graft-tunnel interface. In a certain embodiment, the stem cells are selected as allogenic or autologous stem cells.

As the healing process takes several weeks or months it may be advantageous to have a retarded or sustained release of the co-administrated desired substance. In an embodiment of the invention, the co-administrated desired substance is embedded in the fibrin II structure for sustained release. To further sustain the release of the co-administered desired substance, anti-fibrinolytic agents may be added to the tissue sealant to prevent degradation of fibrin.

In other embodiments of the invention it is desired to have a fast initial burst release of a desired substance. In this case, a proteolytic enzyme, such as plasmin or trypsin, may be added to the tissue sealant to speed up the degradation process.

### EXAMPLE

A study was conducted to evaluate the enhancing properties of Vivostat^{®} platelet enriched fibrin sealant with regard to graft maturation and healing in addition to its hemostatic and analgesic properties in patients who underwent ACL reconstruction.

### Patients and Methods

An Institutional Review Board (IRB) approval for the study was provided together with the informed consents of patients. The single inclusion criterion was having a total isolated acute ACL injury. The exclusion criteria were having a history of rheumatologic, metabolic or malignant disorder, having any previous ACL or other surgery in the same joint, accompanying posterior cruciate ligament (PCL) and/or posterolateral corner (PLC) injuries and meniscal injury requiring repair or excision.

44 patients were enrolled in the study. All patients were operated within 3 weeks following their initial injury (12.4 days (range 4-22)) after receiving pre-operative rehabilitation to provide quadriceps control for full extension with being able to lock the knee in terminal point and achieve 120 to 140 degrees of painless flexion without any apparent edema. All patients underwent anatomic single tunnel, all-inside ACL reconstruction with only autologous semitendinosus (ST) tendon. Femoral and tibial fixations were performed by using two TightRopes^{®} (Arthrex, Munich, Germany).

Group 1 included 23 patients (5 females, 18 males) who had platelet enriched fibrin sealant application by using Vivostat^{®} system (Vivostat A/S, Allerød, Denmark) during surgery. Group 2 as the control group included 21 patients (4 females, 17 males) who did not receive any platelet enriched fibrin sealant application. In Group 1, 120 ml of blood was derived from the patient to prepare platelet enriched fibrin sealant before the induction of anesthesia and antibiotic prophylaxis in the premedication room. At the end of the ACL reconstruction, knees in group 1 were emptied and platelet enriched fibrin sealant was sprayed using a dedicated application device in the Vivostat^{®} system over the graft body, inside the femoral tunnel, both tendon-tunnel interfaces and to areas where bleeding was expected like posterior to PCL, notch and any bleeding site observed in the setting of dry-arthroscopy.

No tourniquet was used and no drains were placed in any group. In both groups, no external braces were used post-operatively. Partial weight bearing as tolerated with two Canadian crutches and full extension and maximum 90 degrees of flexion were allowed for all patients in the same day following the surgery. All patients had Patient Control Analgesia (PCA) after surgery. None of the patients in both groups were professional athletes and were comparable with regard to their demographic data in terms of number, age, gender.

All patients were observed in the hospital at the night of the operation and evaluated the next day. Patients were discharged after 24 hours and examined for any hemarthrosis requiring aspiration during discharge and at the seventh post-operative day. The amount of intravenous patient controlled analgesia (PCA) drug used was noted for all patients. Patients in both groups had the same post-operative rehabilitation protocol in one center. All patients were applied low molecular weight heparin as deep venous thrombosis prophylaxis for 10 days post-operatively. An MRI of the operated knee at the same center with the same device GE Explorer, GE Healthcare, Milwaukee, USA) was taken at the end of post-operative 5th month. A blinded radiologist evaluated the graft's maturation according to its signal intensity and the presence of synovial fluid at the tunnel-graft interface on the MRIs. The termination of the study was defined as the radiological evaluation at the 5th month. MRI measurements for intensity were performed from patellar tendon (PT), quadriceps tendon (QT), posterior cruciate ligament (PCL), reconstructed ACL femoral and tibial tendon tunnel interfaces and graft's mid-body.

For the statistical analysis, SPSS software (Version 22.0; SPSS Inc, Chicago, IL, USA) was used. For quantitative variables between the two groups, Student's t-test was used. Data are expressed as mean +/- SD (standard deviation). The Chi-square test and Fisher's exact test were used for the analysis of categorical variables where appropriate. A p-value less than 0.05 was considered as statistically significant.

### Results

Total follow-up time of the study population was 5 months as determined at the beginning of the study regarding the study design. In group one, after 24 hours following surgery one patient and at the end of the first post-operative week another patient were detected to have hemarthrosis that needed to be aspirated (8.7%). In group two, 4 patients after 24 hours and 4 patients at the end of the first post-operative week were detected to have hemarthrosis that needed to be aspirated (38%) (p=0.003). Group 1 was detected to have a lower rate of post-operative hemarthrosis with high statistical significance. (Table 1)

A PCA (Abbott Laboratories, Abbott Park, IL, USA) protocol was composed of droperidol (5 mg) and morphine (1 mg/mL) in 0.9% normal saline (100 mL). Initially the machine was programmed to deliver a continuous dose of 0.015 mL/kg/hour and a bolus dose of 0.02 mL/kg/hour, which were then adjusted by PCA nurses to maintain patient satisfaction and pain level. It was detected that group one needed the PCA for 10 hours while group two needed it for 11 hours (p=0.08). No significant difference with regard to PCA durations were detected. (Table 1)

**Table 1: Clinical Results**

| | Group 1 | Group 2 | p-Values |
|---|---|---|---|
| Hemarthrosis at the 24^{th} post-op hour | 1 | 4 | - |
| Hemarthrosis at the end of the 1st week | 1 | 4 | - |
| Total Percentage of Hemarthrosis that needed to be aspirated | 8.7% | 38% | 0.003 |
| Mean Total Narcotic Dosage in the PCA device (ml) | 156.74 | 154.27 | 0.27 |

Before starting to compare the signal intensities of ACL grafts, signal intensities of PT, QT and PCL were compared to see whether they were comparable. The measurements of Group 1 and 2 average signal intensities of PT were 2.07 (range 1.1-3.9) and 2.49 (range 1.1-15.5) respectively (p=0.533), of QT were 2.43 (range 1.1-5.8 and 3.03 (range 0.9-24.2) respectively (p=0.580) and of PCL were 3.17 (range 0.3-11.8) and 2.49 (range 1.1-25.7) respectively (p=0.888). Both groups were found to be comparable with regard to their signal intensities of their intact ligament and tendons on the MRI.

Average signal intensity of the ST grafts at the femoral and tibial tendon tunnel interfaces and graft's mid-body were measured on the MRI. Group 1 was detected to have an average signal intensity of graft of 16.59 (range 2.83-45.83) while group 2 had an average signal intensity of 9.19 (range 1.23-10.86) (p=0.047). The average signal intensities of the proximal femoral ST grafts of group 1 and 2 were 23.98 (range 3.8-107.6) and 11.57 (range 1.3-67.5) respectively (p=0.044). The average signal intensities of the mid-body ST grafts of group 1 and 2 were 13.83 (range 2-38.1) and 9.53 (range 1.2-62.5) respectively (p=0.237). The average signal intensities of the distal tibial ST grafts of group 1 and 2 were 11.98 (range 2.7-42.9) and 6.48 (range 1.1-46.7) respectively (p=0.087) (Table 2). Signal intensities of proximal ST grafts were found to be significantly higher in group 1 where platelet enriched fibrin sealant was applied, while average signal intensities were also higher in group 1 with low statistical significance. While group 1's middle and distal average signal intensities of ST grafts were noted to be higher than group 2, no statistical significance was detected.

Synovial fluid was detected in 1 patient in group 1 (4.3%) and in 3 patients in group 2 (14.3%) (p<0.001). Less synovial fluid was detected in patients who underwent platelet enriched fibrin sealant application by using Vivostat^{®} system with high statistical significance. (Table 2)

**Table 2: Radiographic Results**

| | Group 1 | Group 2 | p-Values |
|---|---|---|---|
| Average Signal Intensity of whole ST Grafts | 16.59 | 9.19 | 0.047 |
| Mean Signal Intensity of Proximal (femoral) ST Grafts | 23.98 | 11.57 | 0.044 |
| Mean Signal Intensity of Mid-body ST Grafts | 13.83 | 9.53 | 0.237 |
| Mean Signal Intensity of Distal ST Grafts | 11.98 | 6.48 | 0.087 |
| Synovial fluid at graft-tunnel interface | 1 (4.3%) | 3 (14.3%) | <0.001 |

The present study used dry arthtroscopy to be sure that platelet enriched fibrin sealant stays on the surface of the grafts after being applied with the special equipment of Vivostat^{®} system and used the MRI scans to conduct a radiologic evaluation about the ligamentization and integration of the semitendinosus grafts. The results reported above confirms that application of platelet enriched fibrin sealant was able to yield superior results as compared to controls at every inspected part (proximal-distal tunnels, mid-body) with a statistical significance at the proximal portion inside the femoral tunnel where it was initiated to be applied and also significantly superior considering the average signal values of the whole grafts. It was also shown that the group with platelet enriched fibrin sealant was noted to have significantly less synovial fluid at the tunnel-graft interface. These results indicate that platelet enriched fibrin sealant applied by the Vivostat^{®} system was able to yield better ligamentization and integration of the ACL grafts. It is currently believed that the fibrin matrix is able to maintain the platelets in the applied position in order to deliver the maximum amount of growth factors to the grafts' surface.

Main strengths of this study is, that it was undertaken in a comparative fashion, where the groups were randomized by computer and the radiographic evaluation was performed by a blinded senior musculoskeletal radiologist. Another strength of this study was that it could be considered as pioneer encouraging the application of autologous platelet enriched fibrin sealant for ACL reconstruction with semitendinosus grafts to achieve a more stable construct.

### Conclusions

This study clearly demonstrates that using autologous platelet enriched fibrin sealant supplied by the Vivostat^{®} system during ACL reconstruction in the setting of dry arthroscopy is a safe and effective method yielding better radiographic outcomes with regard to ligamentization and hyalinization of the semitendinosus grafts providing the opportunity to conduct more stable ACL reconstructions in the future.

Application of platelet enriched fibrin sealant at the end of the arthroscopic ACL reconstruction at the dry setting was able to yield superior graft integration and maturation with significantly lower rates of post-operative hemarthrosis that needed to be aspirated. No difference in post-operative analgesia was observed.

## Claims

1. A tissue sealant for use in an Anterior Cruciate Ligament (ACL) reconstruction, wherein a tunnel in femur and a tunnel in tibia are drilled and a tendon graft is accommodated in the femoral tunnel and the tibial tunnel for connecting the femur and the tibia bones together, the tissue sealant being provided on at least a part of the surface of the tendon graft, said tissue sealant comprising 8 mg/ml or more fibrin and at least one human or recombinant growth factor and/or platelets, and wherein at least a portion of the fibrin is fibrin I monomer at a pH lower than pH 5.

2. The tissue sealant according to claim 1, wherein the tendon graft is selected among the hamstring tendon, the patellar ligament, or the quadriceps tendon.

3. The tissue sealant according to any one of claims 1 or 2, wherein the tendon graft is semitendinosus tendon graft.

4. The tissue sealant according to any one preceding claim, wherein tissue sealant is sprayed on the surface of the tendon graft.

5. The tissue sealant according to claim 4, wherein the tissue sealant further is sprayed posterior to the posterior cruciate ligament (PCL).

6. The tissue sealant according to any one of the preceding claims, wherein the fibrin sealant is derived from mammalian blood.

7. The tissue sealant according to any one preceding claim, wherein the tissue sealant is enriched with platelets.

8. The tissue sealant according to claim 7, wherein the concentration of platelets in the tissue sealant is at least twice the concentration of platelets a patient's blood.

9. The tissue sealant according to any one preceding claim comprising 8 to 50 mg/ml fibrin I, such as 15 to 25 mg/ml.

10. the tissue sealant according to any one preceding claim, wherein the tendon graft is an allograft.

11. the tissue sealant according to any one preceding claim, wherein the one or more recombinant growth factor(s) is bone morphogenic protein (BMP).

## Patentansprüche

1. Gewebekleber zur Verwendung in einer Rekonstruktion eines vorderen Kreuzbandes (ACL), wobei ein Tunnel in Oberschenkelknochen und ein Tunnel in Schienbeinknochen gebohrt werden und ein Sehnentransplantat in dem Oberschenkelknochentunnel und dem Schienbeinknochentunnel untergebracht ist, um den Oberschenkelknochen und den Schienbeinknochen miteinander zu verbinden, wobei der Gewebekleber auf mindestens einem Teil der Oberfläche des Sehnentransplantats bereitgestellt ist, wobei der Gewebekleber 8 mg/ml oder mehr Fibrin und mindestens einen menschlichen oder rekombinanten Wachstumsfaktor und/oder Blutplättchen umfasst, und wobei mindestens ein Teil des Fibrins Fibrin-I-Monomer bei einem pH-Wert von weniger als pH 5 ist.

2. Gewebekleber nach Anspruch 1, wobei das Sehnentransplantat aus der Hamstring-Sehne, dem Patellaligament oder der Quadrizepssehne ausgewählt ist.

3. Gewebekleber nach einem der Ansprüche 1 oder 2, wobei das Sehnentransplantat ein Semitendinosus-Sehnentransplantat ist.

4. Gewebekleber nach einem der vorstehenden Ansprüche, wobei der Gewebekleber auf die Oberfläche des Sehnentransplantats gesprüht wird.

5. Gewebekleber nach Anspruch 4, wobei der Gewebekleber ferner hinter dem hinteren Kreuzband (PCL) aufgesprüht wird.

6. Gewebekleber nach einem der vorstehenden Ansprüche, wobei der Fibrinkleber aus Säugetierblut gewonnen wird.

7. Gewebekleber nach einem der vorstehenden Ansprüche, wobei der Gewebekleber mit Blutplättchen angereichert ist.

8. Gewebekleber nach Anspruch 7, wobei die Konzentration an Blutplättchen in dem Gewebekleber mindestens doppelt so hoch ist wie die Konzentration an Blutplättchen in dem Blut eines Patienten.

9. Gewebekleber nach einem der vorstehenden Ansprüche, umfassend 8 bis 50 mg/ml Fibrin I, wie beispielsweise 15 bis 25 mg/ml.

10. Gewebekleber nach einem der vorstehenden Ansprüche, wobei das Sehnentransplantat ein Allotransplantat ist.

11. Gewebekleber nach einem der vorstehenden Ansprüche, wobei der eine oder die mehreren rekombinanten Wachstumsfaktoren knochenmorphogenes Protein (BMP) ist/sind.

## Revendications

1. Agent de scellement tissulaire destiné à être utilisé dans une reconstruction de ligament croisé antérieur (LCA), dans lequel un tunnel dans le fémur et un tunnel dans le tibia sont forés et une greffe de tendon est logée dans le tunnel fémoral et le tunnel tibial pour relier les os du fémur et du tibia ensemble, l'agent de scellement tissulaire étant prévu sur au moins une partie de la surface de la greffe de tendon, ledit agent de scellement tissulaire comprenant 8 mg/ml ou plus de fibrine et au moins un facteur de croissance humain ou recombinant et/ou des plaquettes, et dans lequel au moins une partie de la fibrine est un monomère de fibrine I à un pH inférieur à pH 5.

2. Agent de scellement tissulaire selon la revendication 1, dans lequel la greffe de tendon est choisie parmi le tendon du jarret, le ligament rotulien ou le tendon du quadriceps.

3. Agent de scellement tissulaire selon l'une quelconque des revendications 1 ou 2, dans lequel la greffe de tendon est une greffe de tendon semi-tendineux.

4. Agent de scellement tissulaire selon l'une quelconque des revendications précédentes, dans lequel l'agent de scellement tissulaire est pulvérisé sur la surface de la greffe de tendon.

5. Agent de scellement tissulaire selon la revendication 4, dans lequel l'agent de scellement tissulaire est en outre pulvérisé derrière le ligament croisé postérieur (LCP).

6. Agent de scellement tissulaire selon l'une quelconque des revendications précédentes, dans lequel l'agent de scellement à base de fibrine est dérivé de sang de mammifère.

7. Agent de scellement tissulaire selon l'une quelconque des revendications précédentes, dans lequel l'agent de scellement tissulaire est enrichi en plaquettes.

8. Agent de scellement tissulaire selon la revendication 7, dans lequel la concentration de plaquettes dans l'agent de scellement tissulaire est au moins deux fois la concentration de plaquettes du sang d'un patient.

9. Agent de scellement tissulaire selon l'une quelconque des revendications précédentes, comprenant 8 à 50 mg/ml de fibrine I, par exemple 15 à 25 mg/ml.

10. Agent de scellement tissulaire selon l'une quelconque des revendications précédentes, dans lequel la greffe de tendon est une allogreffe.

11. Agent de scellement tissulaire selon l'une quelconque des revendications précédentes, dans lequel les un ou plusieurs facteurs de croissance recombinants sont des protéines morphogéniques osseuses (BMP).
